# EUROPÄISCHE PATENTSCHRIFT

(11) **EP 3 247 272 B1**
(45) Veröffentlichungstag und Bekanntmachung des Hinweises auf die Patenterteilung: **15.05.2019**
(21) Anmeldenummer: 16700709.5
(22) Anmeldetag: 14.01.2016
(51) Int. Cl.: A61B 5/18, A61B 5/11, A61B 5/00, A61B 5/0245, A61B 5/021, H03H 17/02, G06F 17/15

(54) **VERFAHREN UND VORRICHTUNG ZUM ERKENNEN DER VERFASSUNG VON FAHRZEUGINSASSEN**
METHOD AND APPARATUS FOR RECOGNISING THE CONDITION OF VEHICLE OCCUPANTS
PROCÉDÉ ET DISPOSITIF DE RECONNAISSANCE DE L'ÉTAT DES OCCUPANTS D'UN VÉHICULE

(30) Priorität: 20.01.2015 DE 102015200756
(43) Veröffentlichungstag der Anmeldung: 29.11.2017
(73) Patentinhaber: Ford Global Technologies, LLC, Dearborn, MI 48126 (US)
(72) Erfinder: ARNDT, Christoph, 57583 Mörlen (DE); GUSSEN, Uwe, 52393 Hürtgenwald (DE); STEFAN, Frederic, 52072 Aachen (DE)
(74) Vertreter: Dörfler, Thomas
(86) Internationale Anmeldenummer: PCT/EP2016/050608
(87) Internationale Veröffentlichungsnummer: WO 2016/116342

(56) Entgegenhaltungen:
- US-A1- 2012 123 279
- JAE HYUK SHIN ET AL: "Heart rate variability analysis using a ballistocardiogram during Valsalva manoeuvre and post exercise;Non-constrained heart rate variability analysis using a ballistocardiogram", PHYSIOLOGICAL MEASUREMENT, INSTITUTE OF PHYSICS PUBLISHING, BRISTOL, GB, Bd. 32, Nr. 8, 8. Juli 2011 (2011-07-08), Seiten 1239-1264, XP020208971, ISSN: 0967-3334, DOI: 10.1088/0967-3334/32/8/015

## Beschreibung

Die Erfindung betrifft ein Verfahren und eine Vorrichtung gemäß den Oberbegriffen der unabhängigen Patentansprüche.

Die US 2012/0123279 A1 offenbart ein Verfahren und eine Vorrichtung mit den Merkmalen des Oberbegriffs von Patentanspruch 1. Die Herzschlagparameter umfassen eine Anzahl von vorgegebenen Herzschlagmustern, die auf vielerlei Arten variiert werden. Jedes einzelne Herzschlagmuster wird mittels einer Kreuzkorrelationsfunktion auf Gleichheit mit einem Messsignal überprüft, und die für jedes Einzelmuster gefundenen Maxima der Kreuzkorrelationsfunktion werden gegeneinander ausgewertet.

Ein Verfahren und eine Vorrichtung zum Erkennen der physischen und/oder psychischen Verfassung eines Fahrzeuginsassen anhand eines BKG(Ballistokardiogramm)-Signals, das mittels eines BKG-Sensors gewonnen wird, sind auch aus der US 2013/0158415 A1 bekannt. Der BKG-Sensor ist hier eine Wheatstone-Messbrücke mit Dehnungsmessstreifen in einem Fahrzeugsitz, die bei den geringen Schwingungsamplituden ein sehr hohes Rauschmaß erwarten lässt. Das damit gewonnene BKG-Signal wird mit einem Muster verglichen, das aus vielen in einer Datenbank gespeicherten Mustern ausgewählt wird. Ein derartiges Verfahren erfordert aber einen großen Rechenaufwand, und es hat sich gezeigt, dass brauchbare Ergebnisse auf diese Weise auch nicht schnell genug erhältlich sind.

Aus der DE 101 26 224 A1 und der EP 1 315 451 B1 ist es bekannt, die physische und/oder psychische Verfassung eines Fahrzeuginsassen anhand von EKG- oder EEG-Signalen zu erkennen, doch müssen die Signale kontaktiv erfasst werden, d. h. über den Hautwiderstand, was aufwendig und fehleranfällig ist und/oder dem Fahrzeuginsassen lästig sein kann.

In der DE 10 2011 113 100 A1 ist ein Verfahren beschrieben, bei dem ein erster BKG-Sensors BKG-Signale eines Fahrzeuginsassen erfaßt. Ein zweiter BKG-Sensor ist schwingungsisoliert im Fahrzeugsitze angeordnet und erzeugt eine Störreferenz für das BKG-Signal des ersten Sensors. Zur Minimierung der Störsignale werden die beiden Signale mittels eines Algorithmus verglichen. Der zweite BKG-Sensor bedeutet jedoch einen erheblichen technischen Mehraufwand.

Aus der US 2011 006 6042 A1 ist eine EKG-Meßverfahren bekannt, bei dem die gemessenen Signale gespeichert und das aktuelle Signal mit den gespeicherten Signalen verglichen wird. Allerdings benötigt dieses Verfahren mehrere EKG-Sensoren und ist daher aufwendig.

In der DE 10 2012 111 859 A1 ist ein Fahrzeugsitz offenbart, der EKG-Sensoren. BKG-Sensoren und PKG-Sensoren aufweist. Zur Überwachung der Herzfrequenz des Fahrgastes wird das Signal des Sensors ausgewertet, welches die höchste Genauigkeit aufweist. Durch die hohe Anzahl der benötigten verschiedenen Sensoren ist diese Einrichtung technisch sehr aufwendig.

Der Erfindung liegt die Aufgabe zu Grunde, die physische und/oder psychische Verfassung eines Fahrzeuginsassen schnell, zuverlässig, mit möglichst wenig Verstärker- und Filteraufwand sowie ohne Störung des Fahrzeuginsassen erkennen zu können.

Diese Aufgabe wird durch ein Verfahren und eine Vorrichtung mit den Merkmalen der unabhängigen Patentansprüche gelöst.

Vorteilhafte Weiterbildungen der Erfindung sind in den abhängigen Patentansprüchen angegeben.

Gemäß der Erfindung ist der BKG-Sensor ein MEM-Sensor, ein mikroelektromechanischer Sensor wie z. B. ein Feder-Masse-System, der Beschleunigungen entweder mittels Kapazitätsänderungen oder piezoresistiv erfasst.

Aus der Zeitschrift EL-Info Elektronik Informationen, Heft 11/2013, Seiten 68-70, ist es zwar bekannt, MEM-Sensoren für die Fernerfassung von Herzfunktionen von Patienten mittels Ballistokardiografie zu verwenden. Demnach soll es mittels nicht näher beschriebener Filteralgorithmen möglich sein, das BKS-Signal aus den Resonanzeffekten eines Bettes und dem Rauschen zu extrahieren, was unter den günstigen Bedingungen eines unbewegten Bettes und eines darin ruhendem Patienten realistisch sein kann, aber kaum in der extrem verrauschten Situation in einem fahrenden Fahrzeug.

Die Erfinder haben herausgefunden, dass man mit einem BKG-Signal, dass mittels eines oder mehrerer MEM-Sensoren in einem Fahrzeug gewonnenen wird, bessere, stabilere und schnellere Ergebnisse erzielt, wenn man als erstes in einem Optimalfilter, auch Matched-Filter oder Korrelationsfilter genannt, eine Kreuzkorrelation (Faltung) des BKG-Signals mit Herzschlagparametern durchführt, welche eine Anzahl von Herzschlagmustern sind oder umfassen, die durch Frequenzvariation eines einzigen vorgegebenen Herzschlagmusters innerhalb natürlicher Herzschlag-Grenzen generiert werden, um ein Maximum der Kreuzkorrelationsfunktion zu finden, und als zweites in einer solchermaßen gefundenen Kreuzkorrelationsfunktion wahrscheinliche Peaks (d. h., lokale Maxima) lokalisiert und daraus die Herzfrequenz berechnet.

Das Optimalfilter stellt somit ein zeitvariantes Optimalfilter dar, das über Parameter wie z. B. die Signallänge und Herzfrequenz auf die im Rauschen des Sensorsignals zu suchenden Muster abgestimmt werden kann. Diese Abstimmung erreicht man über die Betrachtung des Maximums der Kreuzkorrelationsfunktion. Variationen in den Parametern können in eingeschwungenem Zustand (Parameter für einen Zeitpunkt fixiert) aus den gefilterten Resultaten direkt übernommen werden.

In einer bevorzugten Ausführungsform wird das Maximum der Kreuzkorrelationsfunktion durch Kurzzeitintervall-Kreuzkorrelation des BKG-Signals mit den generierten Herzschlagmustern in Bezug auf die Variationsfrequenz gefunden.

Gemäß der vorliegenden Erfindung wird nicht in einer großen Datenbank gesucht und dann nochmals adaptiert, sondern
es wird ein vorgegebenes Signal über einen Parameterraum variiert, bis die Peak-Amplitude optimiert ist.

Um die Rechenleistung noch mehr zu vermindern, kann das BKG-Signal einer adaptiven, von der Länge der Herzschlagmuster abhängigen Fensterfunktion unterzogen werden, nachdem das Maximum der Kreuzkorrelationsfunktion gefunden worden ist und bevor die wahrscheinlichen Peaks lokalisiert werden.

Optional kann zusätzlich eine separate Parameter-Abstimmung zur weiteren Optimierung der Peak-Amplitude durchgeführt werden. In beiden Fällen können die Parameter kontinuierlich adaptiert werden, was bei einer Datenbanksuche von Mustern aufgrund limitierter Größe nicht möglich ist. Die optionale Fensterfunktion und Parameter-Abstimmung wirken als Filter, welche falsch erkannte Peaks beseitigen.

Der BKG-Sensor muss in der Lage sein, die Schwingungen in der Hauptschlagader eines Fahrzeuginsassen zu erfassen, die über dessen Körper übertragen werden. Dazu ist der BKG-Sensor vorzugsweise in einem Fahrzeugsitz eingebaut, der in der Regel der Fahrersitz ist, doch könnte er sich möglicherweise auch woanders befinden, z. B. in einen Lenkrad. Außerdem können zusätzlich zu dem Fahrersitz weitere Fahrzeugsitze mit BKG-Sensoren ausgerüstet sein, um die Verfassung der darauf sitzenden Personen zu überwachen.

Als Parameter zum Abschätzen der physischen und/oder psychischen Verfassung eines Fahrzeuginsassen kann mittels des Verfahrens nicht nur dessen Herzfrequenz, sondern auch dessen Blutdruck ermittelt werden, insbesondere weil die Peak-Amplituden mit dem Blutdruck korreliert sind, wobei aber auch ein gewisser Zusammenhang zwischen Herzfrequenz und Blutdruck besteht.

Die Erfindung ermöglicht eine schnelle, zuverlässige und wenig aufwändige Überwachung der physischen und/oder psychischen Verfassung von Fahrzeuginsassen, welche diese in keiner Weise stört und welche es ermöglicht, z. B. einen Fahrer im Falle von Müdigkeit, Stress, Krankheit, medikamentösen Nebenwirkungen, allergischen Schocks oder Körperdehydrierung, zu warnen und ggf. Gegenmaßnahmen zu empfehlen, z. B. einen Stopp einzulegen oder auch per Funk einen Notfalldienstleister zu informieren. Dazu kann die Erfindung mit anderen Verfahren zum Erkennen der physischen und/oder psychischen Verfassung von Fahrern kombiniert werden, z. B. solchen, die Fahrerreaktionen auf bestimmte Verkehrssituationen auswerten.

Zum Kalibrieren eines Systems, in dem das Verfahren durchgeführt wird, kann es derart auf unterschiedliche Dämpfungs- und Steifigkeitsparameter eines Fahrzeugsitzes abgestimmt werden, dass unterschiedliche Sitzdämpfungs- und -stützkoeffizienten erkannt, gespeichert und berücksichtigt werden. Daher kann das Verfahren leicht in beliebigen Fahrzeugen durchgeführt werden, ganz unabhängig von der jeweiligen Sitzkonstruktion (Dämpfungsstruktur aufgrund von unterschiedlichen Materialien; unterschiedliche Sitzabstimmung wie z. B. Sport-Modus, Komfort-Modus, usw.; oder sogar wenn sich Sitzparameter vorübergehend ändern, z. B. aufgrund von Sitzheizung oder -kühlung). Somit besitzt die Erfindung eine einfache Portierbarkeit auf unterschiedliche Fahrzeuge und Sitzkonzepte.

Es folgt eine Beschreibung von Ausführungsbeispielen anhand der Zeichnungen. Darin zeigen:
- Figuren 1-6: verschiedene Signale, die im Rahmen der ballistokardiografischen Herzschlagerkennung erfasst, generiert, verarbeitet und/oder berechnet werden; und
- Fig. 7: ein Blockdiagramm zur Erläuterung der ballistokardiografischen Herzschlagerkennung.

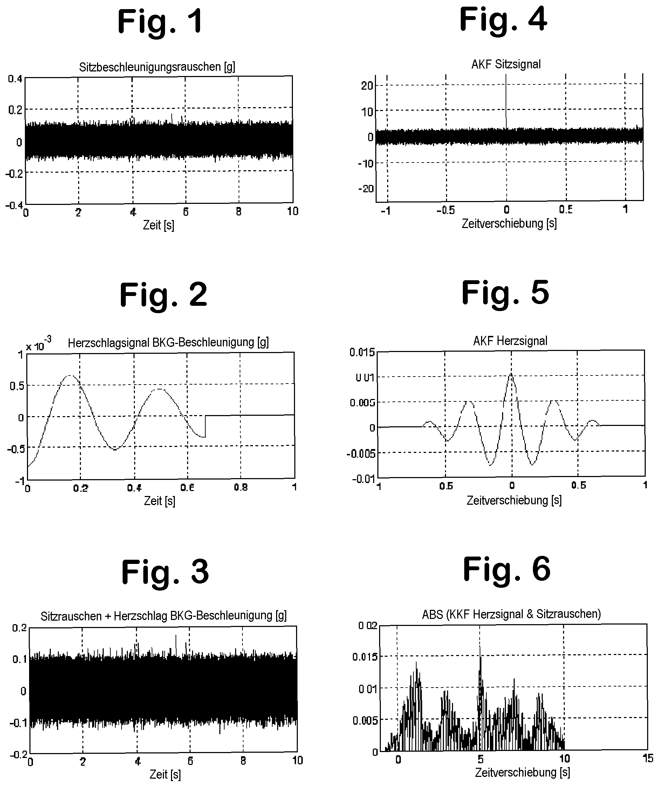

Die in Figuren 1 bis 6 gezeigten Signale sind die folgenden, anhand von realistischen Werten simulierten Signale:
Fig. 1 zeigt ein typisches Sitzbeschleunigungsrauschen in einem Fahrzeugsitz, mit einer mittleren Amplitude von ungefähr 0,1 g während der Fahrt und über eine Zeit von 10 Sekunden.

Fig. 2 zeigt die Amplitude eines Herzschlagsignals, wie es an einem BKG-Sitzsensor ankommt, mit einer maximalen Amplitude von ungefähr 0,0005 g und über eine Zeit von 1 Sekunde. Das Signal hat die Form einer gedämpften Kosinus-Funktion, wobei die Form der Dämpfung hier nicht relevant ist.

Fig. 3 zeigt die vom BKG-Sitzsensor erfasste Summe der Signale von Figuren 1 und 2 über eine Zeit von 10 Sekunden.

Fig. 4 zeigt die Autokorrelationsfunktion des Sitzsignals von Fig. 1, d. h., die Korrelation des Signals mit sich selbst, mit der Zeit- bzw. Phasenverschiebung auf der Abszisse.

Fig. 5 zeigt die Autokorrelationsfunktion des Herzschlagsignals von Fig. 2.

Fig. 6 zeigt den Absolutwert der Kreuzkorrelation der vom BKG-Sitzsensor erfassten Summe aus Sitzrauschen und Herzschlagsignal.

Die Amplitude im Erkennungsprozess hängt von zwei Einflüssen ab, nämlich erstens dem Rauschvorgang bei der Sitzmessung (inhärentes Rauschen sowie durch die Straße und den Fahrer induziertes Rauschen) und zweitens der Länge und Amplitude (Energie) des Herzschlagsignals.

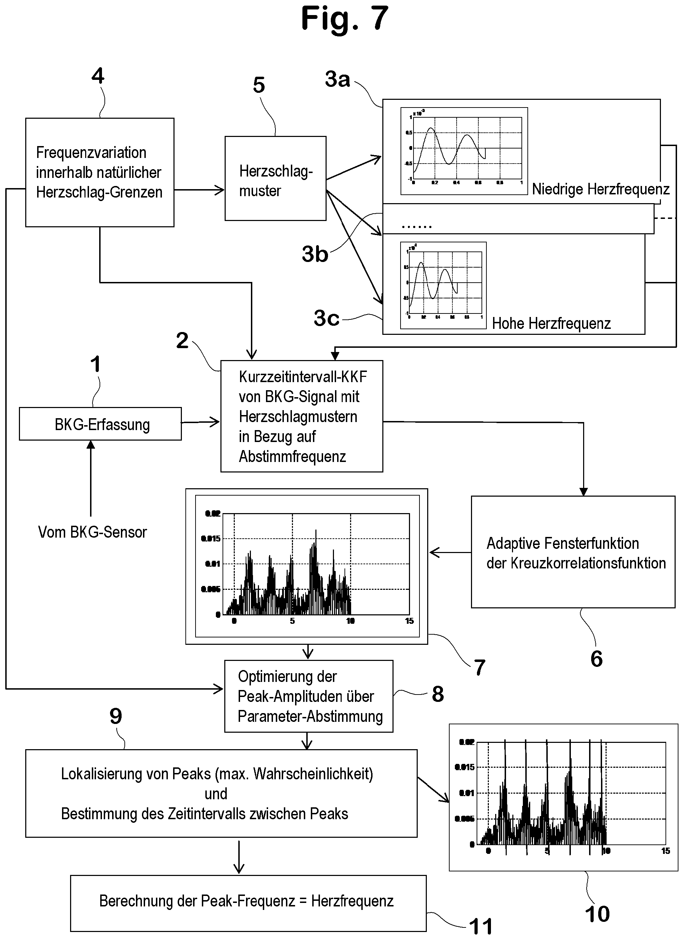

Fig. 7 ist ein Blockdiagramm zur Erläuterung der ballistokardiografischen Herzschlagerkennung in einem Kraftfahrzeug. Im Block 1 wird laufend ein BKG-Signal von einem Sitzsensor erfasst und in Abschnitten, die kurzen Zeitintervallen entsprechen, einem Block 2 zugeführt.

Der Block 2 stellt ein Optimalfilter dar, das eine Kurzzeitintervall-Kreuzkorrelation (KKF) des BKG-Signals mit verschiedenen Herzschlagmustern 3a, ..., 3b, ..., 3c durchführt, die unterschiedlichen Herzfrequenzen entsprechen und die durch Frequenzvariation eines vorgegebenen Herzschlagmusters 5 innerhalb natürlicher Herzschlag-Grenzen generiert werden. Dazu empfängt das Optimalfilter 2 auch die jeweilige Abstimmfrequenz der im Block 4 vorgenommenen Frequenzvariation.

Die im Block 2 erhaltene Kreuzkorrelationsfunktion wird im Block 6 in Abhängigkeit von der Länge des Herzschlagmusters und von dem gemessenen BKG-Signal einer adaptiven Fensterfunktion unterzogen, um den Rechenaufwand zu begrenzen.

Nach Durchführung der adaptiven Fensterfunktion erhält man den Absolutwert der Kreuzkorrelation der gemessenen Summe von Sitzrauschen und Herzschlagsignal, wie im Block 7 sowie in Fig. 6 gezeigt.

Für die im Block 7 gezeigte Signalform wird im Block 8 noch eine separate Parameter-Abstimmung zur Optimierung der Peak-Amplituden durchgeführt, bevor im Block 9 die Peaks in der Signalform lokalisiert werden (nach maximaler Wahrscheinlichkeit) und das Zeitintervall zwischen benachbarten Peaks ermittelt wird, wie im Block 10 veranschaulicht.

Daraus ergibt sich dann im Block 11 die Peak-Frequenz, welche die Herzfrequenz des Fahrzeuginsassen repräsentiert.

Diese Herzfrequenz ist kein glattes Signal und erfordert daher ein weiteres, nicht gezeigtes Filter, um Ausreißer zu eliminieren, die aus Nichterkennung von Peaks in der Kreuzkorrelationsfunktion resultieren. Ein derartiges Filter kann ein Mittelwertfilter oder ein Kalman-Filter mit Restregelung sein, welche beide im Stande sind, untypische Messungen in Echtzeit zu eliminieren.

Anhand der geglätteten Herzfrequenz des Fahrzeuginsassen kann nun auf dessen physische und/oder psychische Verfassung geschlossen werden kann, wie an sich bekannt.

Das oben beschriebene Verfahren enthält die zwei folgenden wesentlichen Verfahrensschritte: Erstens die Frequenzvariation eines vorgegebenen Herzschlagmusters und die Maximierung von Peak-Amplituden mittels Korrelation von unterschiedlichen Herzschlagmustern mit einem gemessenen BKG-Signal; und zweitens Peak-Identifizierung, Peak Lokalisierung und Ermittlung der Herzfrequenz.

## Patentansprüche

1. Verfahren zum Erkennen der physischen und/oder psychischen Verfassung eines Fahrzeuginsassen anhand eines BKG-Signals, das mittels eines im Fahrzeug eingebauten BKG-Sensors gewonnen wird, der ein MEM-Sensor ist, wobei in einem Optimalfilter (2) einer Vorrichtung zum Erkennen der physischen und/ oder psychischen Verfassung eines Fahrzeuginsassen eine Kreuzkorrelation des BKG-Signals mit Herzschlagparametern durchgeführt wird, welche innerhalb vorgegebener Grenzen variiert werden, um ein Maximum der Kreuzkorrelationsfunktion zu finden, und dass in einer solchermaßen gefundenen Kreuzkorrelationsfunktion wahrscheinliche Peaks lokalisiert werden (9) und daraus die Herzfrequenz berechnet wird (11),
**dadurch gekennzeichnet, dass**
die Herzschlagparameter eine Anzahl von Herzschlagmustern sind oder umfassen, die durch Frequenzvariation eines einzigen vorgegebenen Herzschlagmusters innerhalb natürlicher Herzschlag-Grenzen generiert werden.

2. Verfahren nach Anspruch 1,
**dadurch gekennzeichnet, dass**
das Maximum der Kreuzkorrelationsfunktion durch Kurzzeitintervall-Kreuzkorrelation des BKG-Signals mit den generierten Herzschlagmustern gefunden wird.

3. Verfahren nach Anspruch 1 oder 2,
**dadurch gekennzeichnet, dass**
nachdem das Maximum der Kreuzkorrelationsfunktion gefunden worden ist und bevor die wahrscheinlichen Peaks lokalisiert werden, das BKG-Signal einer adaptiven Fensterfunktion unterzogen wird.

4. Verfahren nach einem der vorhergehenden Ansprüche,
**dadurch gekennzeichnet, dass**
nachdem das Maximum der Kreuzkorrelationsfunktion gefunden worden ist bzw. die adaptive Fensterfunktion angewendet worden ist und bevor die wahrscheinlichen Peaks lokalisiert werden, eine separate Parameter-Abstimmung zur Optimierung der Peak-Amplituden durchgeführt wird.

5. Verfahren nach einem der vorhergehenden Ansprüche,
**dadurch gekennzeichnet, dass**
lokalisierte Peaks gefiltert werden, um unerkannte Peaks von der Berechnung der Herzfrequenz auszuschließen.

6. Verfahren nach einem der vorhergehenden Ansprüche,
**dadurch gekennzeichnet, dass**
der BKG-Sensor ein Sitzsensor ist.

7. Verfahren nach einem der vorhergehenden Ansprüche,
**dadurch gekennzeichnet, dass**
mittels des Verfahrens nicht nur die Herzfrequenz, sondern auch der Blutdruck des Fahrzeuginsassen ermittelt wird.

8. Verfahren nach einem der vorhergehenden Ansprüche,
**dadurch gekennzeichnet, dass**
unterschiedliche Sitzdämpfungs- und -stützkoeffizienten erkannt, gespeichert und bei der Durchführung des Verfahrens berücksichtigt werden.

9. Vorrichtung zum Erkennen der physischen und/oder psychischen Verfassung eines Fahrzeuginsassen anhand eines BKG-Signals, das mittels eines im Fahrzeug eingebauten BKG-Sensors gewonnen wird,
**dadurch gekennzeichnet, dass**
die Vorrichtung zur Durchführung des Verfahrens nach einem der vorhergehenden Ansprüche eingerichtet ist.

## Claims

1. Method for recognizing the physical and/or mental condition of a vehicle occupant on the basis of a BCG signal, which is obtained by means of a BCG sensor installed in the vehicle, which BCG sensor is an MEM sensor, wherein a cross-correlation of the BCG signal with heartbeat parameters is carried out in an optimum filter (2) of a device for recognizing the physical and/or mental condition of a vehicle occupant, which heartbeat parameters are varied within predefined limits to find a maximum of the cross-correlation function, and probable peaks are located (9) in a cross-correlation function found in such a manner and the heart rate is calculated therefrom (11),
**characterized in that** the heartbeat parameters are or comprise a number of heartbeat parameters, which are generated by frequency variation of a single predefined heartbeat pattern within natural heartbeat limits.

2. Method according to Claim 1,
**characterized in that** the maximum of the cross-correlation function is found by short-term interval cross-correlation of the BCG signal with the generated heartbeat patterns.

3. Method according to Claim 1 or 2,
**characterized in that** after the maximum of the cross-correlation function has been found and before the probable peaks are located, the BCG signal is subjected to an adaptive window function.

4. Method according to any one of the preceding claims, **characterized in that** after the maximum of the cross-correlation function has been found and/or the adaptive window function has been applied and before the probable peaks are located, a separate parameter adaptation is carried out to optimize the peak amplitudes.

5. Method according to any one of the preceding claims, **characterized in that** located peaks are filtered to exclude unrecognized peaks from the calculation of the heart rate.

6. Method according to any one of the preceding claims, **characterized in that** the BCG sensor is a seat sensor.

7. Method according to any one of the preceding claims, **characterized in that** not only the heart rate, but also the blood pressure of the vehicle occupant is ascertained by means of the method.

8. Method according to any one of the preceding claims, **characterized in that** different seat damping and support coefficients are recognized, stored, and taken into consideration when carrying out the method.

9. Device for recognizing the physical and/or mental condition of a vehicle occupant on the basis of a BCG signal, which is obtained by means of a BCG sensor installed in the vehicle,
**characterized in that** the device is configured to carry out the method according to any one of the preceding claims.

## Revendications

1. Procédé, destiné à identifier la condition physique et/ou psychique d'un occupant de véhicule à l'aide d'un signal BKG, qui est acquis au moyen d'un capteur BKG intégré dans véhicule, qui est un capteur MEM, dans un filtre optimal (2) dans un dispositif destiné à identifier la condition physique et/ou psychique d'un occupant de véhicule étant réalisée une corrélation croisée du signal BKG avec des paramètres de battements cardiaques, que l'on fait varier dans des limites prédéfinies, pour trouver un maximum de la fonction de corrélation croisée, et dans une fonction de corrélation croisée trouvée de la sorte, des pics probables étant localisés (9) et à partir d'eux étant calculée la fréquence cardiaque (11),
**caractérisé en ce que**
les paramètres de battement cardiaque sont ou comprennent une quantité de modèles de battements cardiaques, qui par variation de fréquences d'un unique modèle prédéfini de battements cardiaques sont générés dans des limites naturelles de battements cardiaques.

2. Procédé selon la revendication 1,
**caractérisé en ce que**
le maximum de la fonction de corrélation croisée est trouvée par corrélation croisée à intervalles de courte durée du signal BKG avec les modèles de battements cardiaques générés.

3. Procédé selon la revendication 1 ou 2,
**caractérisé en ce**
**qu'**une fois que le maximum de la fonction de corrélation croisée a été trouvé et avant que les pics probables aient été localisés, le signal BKG est soumis à une fonction de fenêtre adaptative.

4. Procédé selon l'une quelconque des revendications précédentes,
**caractérisé en ce**
**qu'**une fois que le maximum de la fonction de corrélation croisée a été trouvé et la fonction de fenêtre adaptative a été appliquée et avant que les pics probables aient été localisés, une adaptation séparée des paramètres est réalisée pour optimiser les amplitudes de pics.

5. Procédé selon l'une quelconque des revendications précédentes,
**caractérisé en ce que**
des pics localisés sont filtrés pour exclure des pics inconnus du calcul de la fréquence cardiaque.

6. Procédé selon l'une quelconque des revendications précédentes,
**caractérisé en ce que**
le capteur BKG est un capteur de siège.

7. Procédé selon l'une quelconque des revendications précédentes,
**caractérisé en ce**
**qu'**au moyen du procédé, non seulement la fréquence cardiaque, mais également la tension artérielle de l'occupant du véhicule est déterminée.

8. Procédé selon l'une quelconque des revendications précédentes,
**caractérisé en ce que**
des différents coefficients d'amortissement et de soutien du siège sont identifiés, mémorisés et pris en considération lors de la réalisation du procédé.

9. Dispositif, destiné à identifier la condition physique et/ou psychique d'un occupant de véhicule à l'aide d'un signal BKG, qui est acquis au moyen d'un capteur BKG intégré dans le véhicule,
**caractérisé en ce que**
le dispositif est aménagé pour la réalisation du procédé selon l'une quelconque des revendications précédentes.
